(19) Europäisches Patentamt
European Patent Office
Office européen des brevets

(11) EP 1 059 276 A1

(12) **EUROPEAN PATENT APPLICATION**

(43) Date of publication:
**13.12.2000 Bulletin 2000/50**

(51) Int. Cl.$^7$: **C07C 5/42**, B01J 27/188

(21) Application number: **00304571.3**

(22) Date of filing: **30.05.2000**

(84) Designated Contracting States:
**AT BE CH CY DE DK ES FI FR GB GR IE IT LI LU MC NL PT SE**
Designated Extension States:
**AL LT LV MK RO SI**

(30) Priority: **11.06.1999 US 139054 P**

(71) Applicants:
- **ROHM AND HAAS COMPANY**
  **Philadelphia, Pennsylvania 19106-2399 (US)**
- **Sunoco, Inc. (R&M)**
  **Philadelphia, PA 19103-1699 (US)**

(72) Inventors:
- **Eldredge, Josephine Louise**
  **Norristown, Pennsylvania 19403 (US)**
- **Link, Richard David, III**
  **Levittown, Pennsylvania 19054 (US)**
- **Volpe, Anthony Frank, Jr.**
  **Landsdale, Pennsylvania 19446 (US)**

(74) Representative:
**Buckley, Guy Julian**
**ROHM AND HAAS (UK) LTD.**
**European Operations Patent Department**
**Lennig House**
**2 Mason's Avenue**
**Croydon CR9 3NB (GB)**

(54) **Process for preparing alkenes**

(57) Alkanes are converted to alkenes by contacting an alkane with an oxidizing agent and a heteropolyacid supported on a polyoxometallate salt. Also disclosed are catalyst compositions of heteropolyacids supported on polyoxometallate salts and methods of preparing these catalyst compositions.

EP 1 059 276 A1

## Description

### Background of the Invention

This invention relates generally to a process for the preparation of alkenes from alkanes. In particular, this invention relates to a process for preparing alkenes using catalyst compositions comprising heteropolyacids supported on polyoxometallate salts.

Heteropolyacids and polyoxometallates are well known as catalysts in a variety of applications, such as the oxidation of propylene and isobutylene to acrylic and methacrylic acids, the oxidation of aromatic hydrocarbons, olefin polymerization, olefin epoxidation, hydrodesulfurization processes, and the oxidation of alkanes to alcohols. For example, US 5,705,685 (Lyons et al.) discloses the use of heteropolyacids and polyoxometallates for the oxidation of alkanes to unsaturated carboxylic acids.

Acrylic acid and methacrylic acid are of great industrial importance, for example, as monomers for a wide variety of polymers. Acrylic acid is generally prepared from propylene or acrolein and methacrylic acid is generally prepared from isobutylene or methacrolein. A number of attempts have been made to produce acrylic acid directly from propane. For example, US Pat. Application No. 09/002,816 (Lyons et al.) discloses the conversion of propane to acrylic acid using a heteropolyacid on a polyoxometallate support. Also, Japanese Patent Application No. 6-218286 (Jinbo et al.) discloses the conversion of propane to acrolein and/or acrylic acid catalyzed by extra-framework metal substituted heteropolyacids; i.e., cation-exchanged heteropolyacids. Non-framework substituted polyoxometallates and heteropolyacids are also known in the art as catalysts for the direct production of methacrylic acid and methacrolein from the oxidation of isobutane. While these direct methods of producing acrylic acid or methacrylic acid are desirable, they have not yet been successful on a commercial scale. Thus, propylene is still the predominant starting material for the production of acrylic acid.

Propylene is also used commercially in a variety of other applications, most importantly, as a monomer for various polymers. Propylene is typically obtained as cracking gas in the cracking of petroleum hydrocarbons or by thermal dehydrogenation of propane at high temperature, which has many practical disadvantages. For example, thermal dehydrogenation of alkanes typically requires high temperatures, such as 650° C, and has a theoretical maximum of conversion of less than 100%, such as 65% for the conversion of propane to propylene. Selective oxidative dehydrogenation ("ODH") of alkanes to alkenes, such as propane to propylene, is known. Such ODH processes are exothermic, and thus run at a lower temperature than thermal dehydrogenations, however the yields from such ODH are typically very low. Therefore, propylene is several times more expensive than propane.

Mizuno, et. al., Appl. Catal. A: General, 146 (1996), p. L249, disclose propane oxidative dehydrogenation to propylene using heteropolyacids. However, this process is run at high propane and oxygen levels which are impractical in commercial applications. For example, the high oxygen levels require the use of oxygen as the oxidizing agent instead of air. Also, the catalysts disclosed in this paper are prepared by a bulk method which limits the heteropolyacid/polyoxometallate combinations that may be used. This paper does not recognize that alkene formation can be controlled by controlling the amount of the heteropolyacid on the surface of the polyoxometallate support.

Thus, there is a continuing need for a process of preparing alkenes, such as propylene and isobutylene, at a reduced temperature and cost under commercially applicable conditions.

### Summary of the Invention

The process of the present invention provides such a process for the conversion of alkanes to alkenes. The advantages of the process according to the invention are that the higher catalytic activities of the catalysts used in the process allow the process to be carried out at lower temperatures, typically at less than 400° C, than those currently used for oxidative dehydrogenation, while having high reaction rates, yields and selectivities. Also, the present process can avoid conditions requiring high alkane excess. These advantages make the process more attractive than known processes for practical use and potential commercial interest.

In one aspect, the present invention is directed to a process for conversion of alkanes to alkenes including contacting an alkane with an oxidizing agent and a heteropolyacid on a polyoxometallate support; wherein the heteropolyacid has the formula

$$H_{(e'-bz')}G_b(X_{k'}M_{m'-x'}M^1_{x'}M^2_{n'}O_{y'})^{-e'} \qquad (I)$$

wherein G is an element selected from Groups 1-16 or an oxy ion thereof; X is an element selected from Groups 3-16; M = molybdenum, tungsten or a combination thereof; $M^1$ = vanadium; $M^2$ is a transition metal different from M and $M^1$; z' = the charge on G; b = 0 to 12; k' = 1 to 5; m' = 5 to 20; x' = 0 to 6; n' = 0 to 3; y' = 18 to 62; and e' is the charge of the polyoxometallate anion;

wherein the polyoxometallate support has the formula

$$C_aH_{(e-az)}(X_kM_{m-x}M^1{}_xM^2{}_nO_y)^{-e} \quad (II)$$

wherein C is a cation selected from potassium, rubidium, cesium, magnesium, calcium, strontium, barium, transition metal, actinide metal, lanthanide metal, metal oxy ion, ammonium, tetraalkylammonium, pyridinium, quinolinium, protonated aromatic amines, protonated aliphatic amines or mixtures thereof; X is an element selected from Groups 3-16; M is as defined above; $M^1$ = vanadium; $M^2$ is a transition metal different from M and $M^1$; z = the charge on C; k = 1 to 5; m = 5 to 20; n = 0 to 3; x = 0 to 6; y = 18 to 62; and e is the charge of the polyoxometallate anion; and

wherein the heteropolyacid is present in an amount such that less than 100% of the surface area of the polyoxometallate is covered.

In a second aspect, the present invention is also directed to a catalyst composition for the conversion of alkanes to alkenes including a heteropolyacid on a polyoxometallate support; wherein the heteropolyacid has the formula

$$H_{(e'-bz')}G_b(X_{k'}M_{m'-x'}M^1{}_{x'}M^2{}_{n'}O_{y'})^{-e'} \quad (I)$$

wherein G is an element selected from Groups 1-16 or an oxy ion thereof; X is an element selected from Groups 3-16; M = molybdenum, tungsten or a combination thereof; $M^1$ = vanadium; $M^2$ is a transition metal different from M and $M^1$; z' = the charge on G; b =0 to 12; k' = 1 to 5; m' = 5 to 20; x' = 0 to 6; n' = 0 to 3; y' = 18 to 62; and e' is the charge of the polyoxometallate anion;

wherein the polyoxometallate support has the formula

$$C_aH_{(e-az)}(X_kM_{m-x}M^1{}_xM^2{}_nO_y)^{-e} \quad (II)$$

wherein C is a cation selected from potassium, rubidium, cesium, magnesium, calcium, strontium, barium, transition metal, actinide metal, lanthanide metal, metal oxy ion, ammonium, tetraalkylammonium, pyridinium, quinolinium, protonated aromatic amines; protonated aliphatic amines or mixtures thereof; X is an element selected from Groups 3-16; M is as defined above; $M^1$ = vanadium; $M^2$ is a transition metal different from M and $M^1$; z = the charge on C; k = 1 to 5; m = 5 to 20; n = 0 to 3; x = 0 to 6; y = 18 to 62; and e is the charge of the polyoxometallate anion; and

wherein the heteropolyacid is present in an amount such that less than 100% of the surface area of the polyoxometallate is covered.

In a third aspect, the present invention is directed to a process for preparing a catalyst for the conversion of alkanes to alkenes including combining a heteropolyacid with a polyoxometallate support; wherein the heteropolyacid has the formula

$$H_{(e'-bz')}G_b(X_{k'}M_{m'-x'}M^1{}_{x'}M^2{}_{n'}O_{y'})^{-e'} \quad (I)$$

wherein G is an element selected from Groups 1-16 or an oxy ion thereof; X is an element selected from Groups 3-16; M = molybdenum, tungsten or a combination thereof; $M^1$ = vanadium; $M^2$ is a transition metal different from M and $M^1$; z' = the charge on G; b =0 to 12; k' = 1 to 5; m' = 5 to 20; x' = 0 to 6; n' =0 to 3; y' = 18 to 62; and e' is the charge of the polyoxometallate anion;

wherein the polyoxometallate support has the formula

$$C_aH_{(e-az)}(X_kM_{m-x}M^1{}_xM^2{}_nO_y)^{-e} \quad (II)$$

wherein C is a cation selected from potassium, rubidium, cesium, magnesium, calcium, strontium, barium, transition metal, actinide metal, lanthanide metal, metal oxy ion, ammonium, tetraalkylammonium, pyridinium, quinolinium, protonated aromatic amines, protonated aliphatic amines or mixtures thereof; X is an element selected from Groups 3-16; M is as defined above; $M^1$ = vanadium; $M^2$ is a transition metal different from M and $M^1$; z = the charge on C; k = 1 to 5; m = 5 to 20; n = 0 to 3; x = 0 to 6; y = 18 to 62; and e is the charge of the polyoxometallate anion; and

wherein the heteropolyacid is present in an amount such that less than 100% of the surface area of the polyoxometallate is covered.

In a fourth aspect, the present invention is directed to a process for preparing a catalyst composition including a heteropolyacid on a polyoxometallate support for the conversion of an alkane to an alkene including treating the polyoxometallate with a strong, proton containing acid to provide a heteropolyacid on the surface of the polyoxometallate;wherein the heteropolyacid has the formula

$$H_{(e'-bz')}G_b(X_{k'}M_{m'-x'}M^1{}_{x'}M^2{}_{n'}O_{y'})^{-e'} \quad (I)$$

wherein G is an element selected from Groups 1-16 or an oxy ion thereof; X is an element selected from Groups 3-16; M = molybdenum, tungsten or a combination thereof; $M^1$ = vanadium; $M^2$ is a transition metal different from M and $M^1$; z' = the charge on G; b = 0 to 12; k'= 1 to 5; m' = 5 to 20; x' = 0 to 6; n' = 0 to 3; y' = 18 to 62; and e' is the charge of the polyoxometallate anion;

wherein the polyoxometallate has the formula

$$C_aH_{(e-az)}(X_kM_{m-x}M^1{}_xM^2{}_nO_y)^{-e} \quad (II)$$

wherein C is a cation selected from potassium, rubidium, cesium, magnesium, calcium, strontium, barium, transition metal, actinide metal, lanthanide metal, metal oxy ion, ammonium, tetraalkylammonium, pyridinium, quinolinium, protonated aromatic amines, protonated aliphatic amines or mixtures thereof; X is an element selected from Groups 3-16; M is as defined above; $M^1$ = vanadium; $M^2$ is a transition metal different from M and $M^1$; z = the charge on C; k = 1 to 5; m = 5 to 20; n = 0 to 3; x = 0 to 6; y = 18 to 62; and e is the charge of the polyoxometallate anion; and

wherein the amount of the heteropolyacid is present in an amount such that less than 100% of the surface area of the polyoxometallate is covered.

In a fifth aspect, the present invention is directed to catalyst compositions prepared as described above.

Brief Description of the Drawings

Figure 1 shows the relationship of the improvement in propylene selectivity from propane with increasing average pore diameter for a variety of heteropolyacids supported on polyoxometallates.

Detailed Description of the Invention

As used throughout the specification, the following terms shall have the following meanings, unless the context clearly indicates otherwise.

The term "alkane" includes linear, branched and cyclic alkanes. Likewise, the term "alkene" includes linear, branched and cyclic alkenes. All elements are referred to using the nomenclature recommended by the International Union of Pure and Applied Chemistry ("IUPAC"). As used herein, the term "transition metal" refers to an element of Groups 3-12 and includes the lanthanide and actinide series.

All ratios and amounts are by weight, unless otherwise noted. All numerical ranges are inclusive, unless otherwise noted. The following abbreviations are used throughout the specification: C = Centigrade; AA = acrylic acid; HPA = heteropolyacid; POM = polyoxometallate; g = gram; N = normal; mol = mole; L = liter; m = meter; Å = angstrom; and ml = milliliter.

The polyoxometallates and heteropolyacids of the present invention consist of a polyhedral cage structure or framework bearing a negative charge (e.g., $[PW_{12}O_{40}]^{-3}$) which is balanced by cations that are external to the cage. When at least one of the cations is a proton, the compound is a heteropolyacid ("HPA") (e.g., $H_3[PW_{12}O_{40}]$ and $H(VO)[PMo_{12}O_{40}]$). When none of the cations are protons, but are metals such as an alkali metal, potassium, sodium, cesium or lithium, as in $K_3PW_{12}O_{40}$, or ammonium, as in $(NH_4)_3PW_{12}O_{40}$, the compound is a polyoxometallate ("POM"). When a polyoxometallate is combined with an additional acid, for example, sulfuric acid, the resulting mixture is included in the term heteropolyacid. Polyoxoanion describes the anionic cage-like portion of the compound, e.g. $[PW_{12}O_{40}]^{-3}$.

Heteropolyacids and polyoxometallates are cage-like structures with a primary, generally centrally located atom(s) surrounded by a cage framework, which framework contains a plurality of metal atoms, the same or different, bonded to oxygen atoms. The central element of heteropolyacids and polyoxometallates is different from metal atoms of the framework and is sometimes referred to as the "hetero" element or atom; the condensed coordination elements are referred to as the "framework" elements or metals, and are ordinarily transition metals. The majority of heteropolyacids and polyoxometallates have a centrally located heteroatom ("X") usually bonded in a tetrahedral fashion though four oxygen atoms to the "framework" metals ("M"). The framework metals, in turn, (i) are usually bonded to the central atom in an octahedral fashion though oxygens ("O"), and (ii) are bonded to four other framework metals through oxygen atoms and (iii) have a sixth non-bridging oxygen atom known as the "terminal oxygen" atom. This is illustrated by Formula (III).

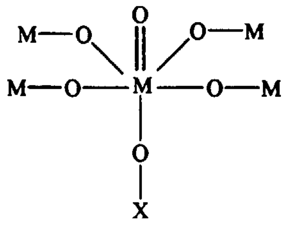

(III)

The principal framework metal, M, is any that has an appropriate cation radius and is a good oxygen pπ-electron acceptor. Typically, the framework metal is selected from molybdenum, tungsten, vanadium, niobium or tantalum. It is preferred that the framework metal is molybdenum, tungsten or vanadium.

Conventional heteropolyacids (and polyoxoanions thereof) can be described by the general formula $H_e(X_kM_nO_y)^{-e}$. In this formula, X, the central atom, is typically a Group 3-16 element, and preferably a Group 13-16 element. Suitable Group 13-16 elements include, but are not limited to: phosphorus, antimony, silicon and boron. It is preferred that the central atom, X, is phosphorus. The subscript "k" is typically from 1 to 5, and preferably 1 or 2. M is typically molybdenum, tungsten, or vanadium. The subscript "n" is typically from 5 to 20. The subscript "y" is typically from 18 to 62, and preferably about 40 to 62. The notation "e" is the negative charge on the $(X_kM_nO_y)$ polyoxoanion and will vary from case to case, but "e" is always the number of protons needed to balance the formula. In a typical Keggin heteropolyacid, k=1, n=12 and y=40 as in $H_3PMo_{12}O_{40}$ and the polyoxometallate $K_4PW_{11}VO_{40}$. In a typical Dawson heteropolyacid, k=2, n=18 and y=62 as in $H_6P_2Mo_{18}O_{62}$ and the polyoxometallate $K_6P_2W_{17}VO_{62}$.

Heteropolyacids and polyoxometallates are known to exist in a variety of structures including the Keggin, Dawson and Anderson structures. These different structures correspond to the specific geometry of particular heteropolyacid compositions and vary according to the coordination chemistry and atomic radii of the metals present. Any of these structures, or mixtures thereof, are suitable for use in the present invention.

Framework-substituted heteropolyacids and polyoxometallates are also useful in the present invention. These compounds are heteropolyacids and polyoxometallates where certain framework atoms M (and the oxygen atoms doubly bonded to them) are replaced with transition metals. The substitution may, for example, be monosubstitution, random- or regio-disubstitution, random-or regio-trisubstitution, or higher substitutions, all of which produce effective compositions for use as the supported heteropolyacid and the polyoxometallate support in the process of the present invention. The catalysts may be further promoted by a variety of means described below. The present invention encompasses unsubstituted and substituted heteropolyacids supported on salts of unsubstituted and substituted polyoxometallates.

A typical heteropolyacid useful in making the framework-substituted compositions has the formula $H_3PMo_{12}O_{40}$. When three Mo=O units are replaced with, e.g. non (Fe), the resulting framework substituted heteropolyacid has the formula $H_6PMo_9Fe_3O_{37}$. Thus, the general formula of the regioselectively framework-substituted heteropolyacids described above becomes:

$$H_e(X_kM_nM^1{}_mO_y)^{-e}$$

wherein k is from 1 to 5, n is from 5 to 19, m is from 1 to 6 and y is from 18 to 62. In this formula, $M^1$ comprises one or more of zinc or any of the transition metals, namely the Group 3-10 metals of the periodic table. Preferably the transition metal is an element selected from Groups 8-10 or the first row of Groups 4-7, such as, but not limited to: iron, cobalt, nickel, ruthenium, rhodium, palladium, osmium, iridium, platinum (Groups 8-10) or titanium, vanadium, chromium, manganese (Groups 4-7, first row). Among the more preferred $M^1$ metals are iron, manganese, vanadium and combinations of nickel and iron or other transition metal. The three $M^1$ atoms do not have to be the same. However, the three $M^1$ atoms must be different from the three M atoms replaced.

By substituting oxidation-active metals, such as iron, for metals, such as molybdenum, in certain Keggin or Dawson structures, superior catalysts for the direct oxidation of alkanes to alkenes, such as, propylene or isobutylene, may be obtained. By superior catalysts are meant catalysts that have increased conversion, yield and/or selectivity for the conversions of alkanes to alkenes. Such oxidation-active metal substitution may be framework or extra-framework, i.e. outside the framework. When such oxidation-active metals are substituted into the heteropolyacids, they may be a substitute for protons.

The heteropolyacids useful in the present invention are typically soluble in water and polar organic solvents, such as acetonitrile and alcohols, such as methanol. Such heteropolyacids are of the formula

$$H_{(e'-bz')}G_b(X_{k'}M_{m'-x'}M^1_{x'}M^2_{n'}O_{y'})^{-e'} \quad (I)$$

wherein G is an element selected from Groups 1-16 or an oxy ion thereof; X is an element selected from Groups 3-16; M = molybdenum, tungsten or a combination thereof; $M^1$ = vanadium; $M^2$ is transition metal different from M and $M^1$; z' = the charge on G; b = 0 to 12; k' = 1 to 5; m' = 5 to 20; x' = 0 to 6; n' = 0 to 3; y' = 18 to 62; and e' is the charge of the polyoxometallate anion. Suitable elements for G include, but are not limited to: titanium, zirconium, hafnium, vanadium, niobium, tantalum, chromium, molybdenum, tungsten, manganese, rhenium, iron, cobalt, nickel, ruthenium, rhodium, palladium, osmium, iridium, platinum, copper, zinc, or combinations thereof. Suitable oxy anions of elements for G include, but are not limited to: titanyl, vanadyl, niobyl, or combinations thereof. Suitable transition elements for X include, but are not limited to: phosphorus, silicon, gallium, aluminum, arsenic, germanium, boron, cobalt, cerium, praseodymium, uranium, thorium or mixtures thereof. Suitable transition elements for $M^2$ include, but are not limited to: titanium, zirconium, hafnium, vanadium, niobium, tantalum, chromium, molybdenum, tungsten, manganese, rhenium, iron, cobalt, nickel, ruthenium, rhodium, palladium, osmium, iridium, platinum, copper, zinc or combinations thereof. It is preferred that when M is a combination of molybdenum and tungsten and the compound is a Keggin ion, x' = 0. It is also preferred that when M is molybdenum and the compound is a Keggin ion, x' = 0 to 3. It is further preferred that when M is tungsten and the compound is a Keggin ion, x' = 0 to 6.

In the above formula (e' - bz') describes the number of protons ("$H^+$") present in the heteropolyacid component of the catalyst. At a minimum, (e' - bz') is preferably greater than or equal to 0.1. In one embodiment of the invention, (e' - bz') is greater than or equal to 0.5, in another it is greater than or equal to 1. In some embodiments, bz' equals zero and the number of protons in the HPA is e'. In another embodiment, (e' - bz') is formally 0 and the protons are added by treating the system with another acid, such as sulfuric acid. Suitable heteropolyacids useful in the present invention include, but are not limited to: $H_3PMo_{12}O_{40}$, $H_3PW_{12}O_{40}$, $(VO)_{1.5}PMo_{12}O_{40}$, $(VO)_{1.5}PW_{12}O_{40}$, $(TiO)_{1.5}PMo_{12}O_{40}$, $H(VO)PMo_{12}O_{40}$, $H(VO)PW_{12}O_{40}$, $H_6PV_3Mo_9O_{40}$, $H_5PV_2Mo_{10}O_{40}$, $H_5PV_2W_{10}O_{40}$, $H_4PVMo_{11}O_{40}$, $H_4PVW_{11}O_{40}$, $RhPMo_{12}O_{40}$, $BiPMo_{12}O_{40}$, $HCrPVMo_{11}O_{40}$, $HBiPVMo_{11}O_{40}$, or combinations thereof. It is preferred that the heteropolyacid is $H_3PMo_{12}O_{40}$, $H_3PW_{12}O_{40}$, $(VO)_{1.5}PMo_{12}O_{40}$, $H(VO)PMo_{12}O_{40}$, $H_5PV_2Mo_{10}O_{40}$, $H_4PVMo_{11}O_{40}$, $RhPMo_{12}O_{40}$, $HCrPVMo_{11}O_{40}$, or $HBiPVMo_{11}O_{40}$. It is more preferred that the heteropolyacid is $H_3PMo_{12}O_{40}$ or $H_3PW_{12}O_{40}$. When the heteropolyacid is, for example, $(VO)_{1.5}PMo_{12}O_{40}$, $(TiO)_{1.5}PMo_{12}O_{40}$, $RhPMo_{12}O_{40}$, or $BiPMo_{12}O_{40}$, the necessary acid typically comes from a separate acid source, such as sulfuric acid in the $VOSO_4$ used to make $(VO)_{1.5}PMo_{12}O_{40}$. Such amount of acid is sufficient for the present invention.

The polyoxometallates useful as supports in the present invention are typically water insoluble and are those of the formula

$$C_aH_{(e-az)}(X_kM_{m-x}M^1_xM^2_nO_y)^{-e} \quad (II)$$

wherein C is a cation selected from potassium, rubidium, cesium, magnesium, calcium, strontium, barium, transition metal, actinide metal, lanthanide metal, metal oxy ion, ammonium, tetraalkylammonium, pyridinium, quinolinium, protonated aromatic amines, protonated aliphatic amines or mixtures thereof; X is an element selected from Groups 3-16; M, the first framework metal, is molybdenum, tungsten or a combination thereof; $M^1$, the second framework metal, is substituted for the first framework metal and is vanadium; $M^2$, the third framework metal, is different from M and $M^1$ and is independently transition metal; z' = the charge on C; k = 1 to 5; m = 5 to 20; n = 0 to 3; x = 0 to 6; y = 18 to 62; and e' is the charge of the polyoxometallate anion. Suitable transition metals useful for C include, but are not limited to: vanadium, chromium, lanthanum, manganese, iron, cobalt, ruthenium, copper and the like. Suitable metal oxy ions useful for C include, but are not limited to: oxy ions of vanadium, oxy ions of chromium, oxy ions of uranium, and the like. It is preferred that C is potassium, rubidium, cesium, magnesium, calcium, strontium, barium, lanthanum, ammonium, tetraalkylammonium, pyridinium, quinolinium, protonated aromatic amines, protonated aliphatic amines, or mixtures thereof, and more preferably cesium. Suitable transition elements for X are those useful as X in the heteropolyacids described above. Suitable transition elements useful for $M^2$ are those described above for the heteropolyacids. When M is molybdenum and the compound is a Keggin ion, it is preferred that x = 0 to 3. When M is tungsten and the compound is a Keggin ion, it is preferred that x = 0 to 6. When "az" equals "e", there are no protons present in the polyoxometallate support, which is preferred.

Suitable polyoxometallate supports useful in the present invention include wide pore salts, for example wide pore cesium salts of the various substituted polyoxometallates described in US Ser. No. 08/565,206, herein incorporated by reference to the extent it teaches the preparation of such salts. It is preferred that the polyoxometallate support is $Cs_{3+x}(PM_{12-x}V_xO_{4O})$, where x = 0 to 3 and M = molybdenum or tungsten. Suitable preferred polyoxometallate supports include $Cs_3(PMo_{12}O_{40})$, $Cs_4(PMo_{11}VO_{40})$, $Cs_5(PMo_{10}V_2O_{40})$, $Cs_6(PMo_9V_3O_{40})$, $Cs_3(PW_{12}O_{40})$, $Cs_4(PW_{11}VO_{40})$, $Cs_5(PW_{10}V_2O_{40})$, $Cs_6(PW_9V_3O_{40})$, or combinations thereof. It is more preferred that the polyoxometallate support is $Cs_3(PMo_{12}O_{40})$, $Cs_3(PW_{12}O_{40})$ or combinations thereof.

The polyoxometallate supports useful in the present invention may have large (i.e. wide) or small pores or a

mixture of pore sizes. It is preferred that the polyoxometallate support has large pores. For example, the polyoxometallate support preferably has pore volumes in the range from 0.01 to 0.25 ml/g and a pore size distribution in which more than approximately 60% of the pore volume is due to pores having a median pore radius of greater than or equal to approximately 75 Å, preferably greater than or equal to approximately 100 Å, more preferably greater than or equal to approximately 150 Å, still more preferably greater than or equal to approximately 200 Å. More preferably, the support has pore volumes in the range from 0.05 to 0.25 ml/g and a pore size distribution in which more than approximately 60% of the pore volume is due to pores having a median pore radius of greater than or equal to approximately 75 Å. In a preferred embodiment, the support material has pore volumes in the range from 0.01 to 0.25 ml/g and a pore size distribution in which more than approximately 80% of the pore volume is due to pores having a median pore radius of greater than or equal to approximately 200 Å; more preferably, the support material has pore volumes greater than 0.15 ml/g and a pore size distribution in which more than approximately 80% of the pore volume is due to pores having a median pore radius of greater than or equal to approximately 200 Å.

In a more preferred embodiment, the pores in the support have pore radii of greater than 75 Å and pore volumes greater than 0.05 ml/g; more preferably, the pore radii are greater than 100 Å, and independently, the pore volumes are greater than 0.1 ml/g. It has been found that supports with pore volumes greater than 0.02 ml/g result in catalysts with superior catalytic performance, provided the pores are wide (i.e., radii greater than approximately 75 Å). These polyoxometallates may be further modified by pretreatment with water, such as steam, washing with various solvents, and by formation in the presence of vanadyl acetylacetonate or $VOSO_4$. Such supports may also be ground to modify pore sizes.

Figure 1 shows the relationship of the improvement in propylene selectivity from propane with increasing pore diameter for a variety of catalysts systems: 2 mole% $H_3PMo_{12}O_{40}$ on $Cs_3PMo_{12}O_{40}$ ("triangle"), 2 mole% $H_5PV_2Mo_{10}O_{40}$ on $Cs_3PMo_{12}O_{40}$ ("circle"), 2 mole% $H_3PMo_{12}O_{40}$ on $Cs_3PW_{12}O_{40}$ ("+"), 2 mole% $H_3PW_{12}O_{40}$ on $Cs_3PW_{12}O_{40}$ ("x"). Thus, it can be seem from Figure 1 that propylene selectivity increases with increasing average pore diameter in the catalyst.

The heteropolyacid/polyoxometallate("HPA/POM") catalysts useful in the present invention are those having the formula

$$H_{(e'-bz')}G_b(X_{k'}M_{m'-x'}M^1{}_{n'}M^2{}_{n'}O_{y'})^{-e'}/C_aH_{(e-az)}(X_kM_{m-x}M^1{}_xM^2{}_nO_y)^{-e} \quad (IV)$$

wherein the individual components are as described in formulae (I) and (II) above. It is preferred that the HPA/POM catalyst is $(VO)_{1.5}PMo_{12}O_{40}/Cs_3(PMo_{12}O_{40})$, $(VO)_{1.5}PMo_{12}O_{40}/Cs_3(PW_{12}O_{40})$, $H(VO)PMo_{12}O_{40}/Cs_3(PMo_{12}O_{40})$, $H_5PV_2Mo_{10}O_{40}/Cs_3(PMo_{12}O_{40})$,$H_5PV_2Mo_{10}O_{40}/Cs_3(PW_{12}O_{40})$, $H_4PVMo_{11}O_{40}/Cs_3(PMo_{12}O_{40})$, $H_4PVMo_{11}O_{40}/Cs_3(PW_{12}O_{40})$, $RhPMo_{12}O_{40}/Cs_3(PMo_{12}O_{40})$, $HCrPVMo_{11}O_{40}/Cs_3(PMo_{12}O_{40})$, $HBiPVMo_{11}O_{40}/Cs_3(PMo_{12}O_{40})$, $H_4(PMo_{11}VO_{40})/Cs_3(PMo_{12}O_{40})$, $H_3(PMo_{12}O_{40})/Cs_3(PMo_{12}O_{40})$, $H_3(PMo_{12}O_{40})/Cs_3(PW_{12}O_{40})$, $H_3(PW_{12}O_{40})/Cs_3(PMo_{12}O_{40})$, or $H_3(PW_{12}O_{40})/Cs_3(PW_{12}O_{40})$, and more preferably $H_3(PMo_{12}O_{40})/Cs_3(PMo_{12}O_{40})$, $H_3(PMo_{12}O_{40})/Cs_3(PW_{12}O_{40})$, $H_3(PW_{12}O_{40})/Cs_3(PMo_{12}O_{40})$ or $H_3(PW_{12}O_{40})/Cs_3(PW_{12}O_{40})$.

The polyoxometallate or heteropolyacid component used in the process of the invention may contain second framework metals which have been substituted into the framework thereof, replacing an equivalent number of the first framework metals. Such substituting metals include, but are not limited to: titanium, zirconium, hafnium, vanadium, niobium, tantalum, chromium, molybdenum, tungsten, manganese, rhenium iron, cobalt, nickel, ruthenium, rhodium, palladium, osmium, iridium, platinum, copper, zinc or combinations thereof. The second framework metal, $M^1$, is different from the first framework metal, M. When there are more than one $M^1$ atoms, each $M^1$ is bound through an oxygen atom to another $M^1$.

The atoms which are replaced in such substitution include, but are not limited to: molybdenum, tungsten, vanadium or combinations thereof. The number of framework atoms replaced may be from 1 to 3 or more, and the substituting metals, which are different from the replaced metal, may each be the same metal, for example iron, or may be different from each other, for example two or three different metal atoms; e.g., one iron atom may replace one tungsten atom; two iron atoms may replace two tungsten atoms; three iron atoms may replace three tungsten atoms; two atoms, different from each other, for example iron and cobalt, may replace two tungsten atoms; three atoms, different from each other, for example iron, cobalt and nickel, may replace three tungsten atoms; two atoms of iron and one atom of cobalt may replace three tungsten atoms; and so on. Replacement of three framework atoms of a POM or HPA by three atoms, different from the framework atom, two of which replacing atoms are selected from the group consisting of iron, chromium, manganese or ruthenium, and the third of which is different from the two just referred to and is a transition metal, is disclosed in U.S. 5,091,354 (Lyons et al.).

Examples of such heteropolyacids include $H_6PW_9Fe_3O_{37} \cdot NaN_3$ wherein phosphorus (P) is the heteroatom, tungsten (W) is the first framework metal, and iron (Fe) is the second framework metal; $H_7PW_9Fe_2MO_{37} \cdot NaN_3$, wherein phosphorus (P) is the heteroatom, tungsten (W) is the first framework metal, and iron (Fe) and "M" are the second framework metals, M being variously nickel, manganese, cobalt, zinc; and $H_7PW_9Cr_3O_{37} \cdot NaN_3$, wherein phosphorus (P) is the heteroatom, tungsten (W) is the first framework metal, and chromium (Cr) is the second framework metal. Examples of such heteropolyacids, include $H_3PW_{10}M_2O_{40}$, where M is titanium, zirconium, niobium, tantalum, manganese, iron, cobalt, nickel or copper. These compositions are useful as the POM and/or HPA component of the supported catalysts of the present invention.

The polyoxometallate or the heteropolyacid, or both, may independently comprise (1) at least 6 atoms of a first framework metal or metals comprising molybdenum, tungsten, vanadium or combinations thereof and (2) at least one atom of a second framework metal or metals comprising a transition metal other than molybdenum, tungsten or vanadium. When there is more than one second framework metal, they may comprise a combination of the available transition metals.

In one embodiment, the polyoxometallate and/or the heteropolyacid used in the process of the invention comprises 9 to 11 atoms of a first framework metal selected from the group consisting of molybdenum, tungsten and vanadium, and 1 to 3 atoms of a second framework metal such as titanium, zirconium, vanadium, niobium, tantalum, chromium, molybdenum, tungsten, manganese, rhenium, iron, cobalt, nickel, ruthenium, rhodium, palladium, osmium, iridium, platinum, copper or zinc, which second metal is a transition metal different from the first framework metal. The second framework metals, $M^1$, are site-specific, regioselective substitutions wherein each $M^1$ is bound through an oxygen atom to another $M^1$.

The supported heteropolyacid catalyst may be pretreated with water, such as with steam. Such pretreatment may provide catalysts which give increased alkene production.

The heteropolyacids useful in the present invention are commercially available or may be prepared by a variety of methods known in the art. General syntheses of the polyoxometallates and heteropolyacids useful in the present invention are described in Pope et. al., *Heteropoly and Isopoly Oxometallates*, Springer-Verlag, New York (1983). Typically, heteropolyacids are prepared by dissolving the desired metal oxides in water, adjusting the pH to approximately 1 to 2 with acid, such as hydrochloric acid, to provide the necessary protons, and then evaporating water until the desired heteropolyacid precipitates. As an example, the heteropolyacid $H_3PMo_{12}O_{40}$ can be prepared by combining $Na_2HPO_4$ and $Na_2MoO_4$, adjusting the pH with sulfuric acid, extracting with ether, and crystallizing the resulting heteropolyacid in water. Also, vanadium-substituted heteropolyacids may be prepared according to the method described in V. F. Odyakov, et. al., *Kinetics and Catalysis*, 1995, vol. 36, p. 733. The $Cs_3PMo_{12}O_{40}$ support can be prepared, as further described below, by treating the above heteropolyacid with cesium carbonate and collecting the resulting precipitated product.

The catalysts useful in the process of the present invention may be promoted by various means including preparing the heteropolyacid in the presence of vanadyl acetylacetonate or the like. In addition, exchange of iron or other transition metals, actinide and lanthanide metals, and other groups, G, has been found to promote the activity of the heteropolyacids of the catalysts used in the process of the present invention.

Typically, the polyoxometallate support component of the catalyst may be prepared by adding a soluble salt of the desired cation, for example $Cs_2CO_3$ for a cesium salt support, to the desired soluble heteropolyacid, for example $H_3(PMo_{12}O_{40})$, to form the insoluble polyoxometallate, for example $Cs_3(PMo_{12}O_{40})$. The polyoxometallates may be isolated by any means, such as by filtration or solvent evaporation, and preferably by solvent evaporation. Certain polyoxometallate precipitates may be isolated without need for an evaporation step. However, in many cases the precipitate is very fine and isolation is preferably done by evaporation of water. The desired proportion of the metal oxides may vary somewhat from the theoretical amount required for the desired product.

The salt solution is preferably added slowly into the heteropolyacid solution to precipitate the cation heteropolymetallate salt. The following reaction exemplifies the process:

$$3\ Cs_2CO_3 + 2\ H_3(PMo_{12}O_{40}) \rightarrow 2\ Cs_3(PMo_{12}O_{40}) + 3\ H_2O + 2\ CO_2$$

The precipitation may be performed at an elevated temperature (e.g., 60 - 65° C) and $CO_2$ is evolved during the reaction. The resulting polyoxometallate salt forms a fine suspension in water and may be evaporated to dryness, for example by rotary evaporation, or by heating, such as at 50 to 70 °C or below. The dried material may be calcined/heat treated (e.g., at 300° C).

It has been found that the preparation process can influence the surface area, the pore volume and the pore size distribution ("PSD") of the polyoxometallate salts. For example, slow addition of the cation salt to the heteropolyacid solution results in a material with few small pores and many large pores. In contrast, rapid addition of the cation salt yields a broad PSD with many small pores and some intermediate and large pores. For the present invention, slow addition to form mainly large pores is preferable; for example, at a rate of 2 ml/minute, particularly when using solution concentrations of approximately 0.1 mol/L. More preferably, particularly for the preparation of large quantities of material, the solutions of the cation salt and the heteropolyacid may be added simultaneously to a reaction vessel. The salt solution may have a concentration in the range of from about 0.05 to 1 mol/L, preferably 0.1 to 0.2 mol/L. The heteropolyacid

solution may have a concentration in the range of from about 0.05 to 1 mol/L, preferably 0.1 to 0.2 mol/L, and more preferably 0.1 mol/L.

A further factor influencing the PSD is the temperature of the reaction medium during the precipitation step. Precipitation at room temperature yielded a narrow PSD with a median pore radius of about 90 Å, whereas precipitation at 65° C was found to result in a broader PSD with a greater median pore radius (≥120 Å).

Additionally, it has been found that aging of the slurry containing the polyoxometallate salt, followed by slow evaporation to dryness, is beneficial to the production of large pore materials. Preferably, the slurry is allowed to remain at room temperature or at a temperature in the range from approximately 35° C to 45° C for an extended period of time and is then slowly dried. The aging and drying process may extend for a period of 1 to 3 days or longer. Also, the use of excess cation salt (relative to the stoichiometric amount) promotes formation of the desired large-pore support material. While the support material can be prepared using stoichiometric ratios of starting materials, it is preferred to use a slight excess of the cation salt.

The preparation of heteropolyacids and polyoxometallates with random framework-metal substitution, such as $H_7(PMo_8V_4O_{40})$; $K_6(SiMo_{11}MnO_{39})$ and $K_5(PW_{11}VO_{40})$, is known. For example, $K_5(PW_{11}VO_{40})$ may be prepared by dissolving 45.0 g of 12-tungstophosphoricacid, $H_3(PMo_{12}O_{40})$, in 105 ml of water. With stirring, the pH is adjusted to about 5.2 with potassium bicarbonate. The mixture is then heated to 70 °C and 6.0 g of vanadyl sulfate ($VOSO_4$) in 15 ml water is added. The solution is cooled and potassium chloride is added to precipitate the desired $K_5(PW_{11}VO_{40})$.

The preparation of regiospecific, trilacunary framework-substituted heteropolyacids or polyoxometallates may also be useful in the present invention. Suitable regiospecific, trilacunary framework-substituted heteropolyacids or polyoxometallates are described in US Pat. No. 4,898,989, herein incorporated by reference to the extent it teaches the preparation of such substituted compounds.

The HPAs and POMs useful in the present invention may be prepared separately and then combined or, alternatively, may be prepared together. It is preferred that the HPAs and POMs are prepared separately and then combined. In an alternative embodiment, the catalysts useful in the present invention may be prepared by generating the heteropolyacid in situ on the surface of the polyoxometallate support by treating the polyoxometallate with a strong, proton containing acid. Any strong, proton containing acid is suitable, and preferably an inorganic acid. Suitable acids include, but are not limited to: hydrochloric acid, hydrobromic acid, sulfuric acid and nitric acid.

When the heteropolyacids useful in the present invention are present as a complete surface layer on the polyoxometallate support, i.e. the heteropolyacids cover 100% of the surface area of the support, alkanes are converted to unsaturated carboxylic acids. For example, propane is converted to acrylic acid. It has been surprisingly found that when the heteropolyacids are present in an amount less than a complete layer, i.e. the heteropolyacids cover less than 100% of the surface area of the support, alkanes are converted to alkenes. Thus, any amount of heteropolyacid that is present in an amount such that less than 100% of the surface area of the polyoxometallate support is covered is useful in the process of the present invention. It is preferred that the amount of the heteropolyacid covers 0.5 to 75% of the surface area of the polyoxometallate support, more preferably 1 to 70% of the surface area, and most preferably 3 to 50% of the surface area.

For example, when the polyoxometallate support is $Cs_3PMo_{12}O_{40}$ having a surface area of 80 $m^2$/g, the amount of the heteropolyacid useful in the present invention is less than 20 mol%, and preferably less than 10 mol% (approximately 50% of the surface area covered). When the polyoxometallate support is $Cs_3PW_{12}O_{40}$ having a surface area of 110 $m^2$/g, the amount of the heteropolyacid useful in the present invention is less than 25 mol%, and preferably less than 12 mol% (approximately 50% of the surface area covered).

When the catalyst useful in the present invention is prepared in situ by treating a polyoxometallate with a strong, proton containing acid, any amount of acid may be used as long as the resulting heteropolyacid covers less than 100% of the surface area of the polyoxometallate support.

The catalysts useful in the present invention comprise a heteropolyacid on a polyoxometallate support, as described above. The supported catalyst comprising a heteropolyacid supported the polyoxometallate salt may be prepared, for example, by incipient wetness techniques in which a solution of heteropolyacid is sprayed on a solid support matrix and then dried, or by adding support material to a solution of heteropolyacid and evaporating the solution to dryness, or by dry grinding the heteropolyacid with the polyoxometallate support. The heteropolyacid may be dissolved in water or other solvent, such as acetonitrile. The resulting material is dried and optionally heat-treated, i.e., calcined.

The following process illustrates the catalyst preparation using incipient wetness technique. The desired amount of heteropolyacid is dissolved in solvent (typically water or acetonitrile). After the heteropolyacid, together with precursors of groups G, if desired, are dissolved, the solution is sprayed evenly on the support material and the supported catalyst is dried, for example at 80° C for 8 hours when using water, or 50° C for 8 hours when using acetonitrile. Repeated spraying and drying steps may used to modify dispersion characteristics. The final supported catalyst material may be heat treated. If heat treated, the heat treatment temperature is preferably between 250° C and 450° C, and is not so severe as to damage the catalyst. The heat treatment may be performed, for example, at 275° C for 3 to 6 hours, or at 420° C for 1 to 2 hours.

In one embodiment of the invention, the supported heteropolyacid may be pre-treated with water. The catalyst is prepared by exposure to air saturated with water vapor or steam for approximately 48 hours. The hydrated catalyst may comprise about 5 to 30 weight percent water. This pretreatment of the catalyst by hydration may enhance catalytic activity.

The process of the present invention is useful for converting alkanes to alkenes. The alkanes useful in the present invention may be linear, branched, or cyclic and are any which are gaseous at the temperature of the reaction. It is preferred that the alkanes useful in the present invention are gaseous at 370 °C, and more preferably gaseous at 225° C. Thus, alkanes having 2 to 20 carbon atoms may be used successfully in the present process. The alkanes may be single compounds or mixtures of compounds. The purity of the alkanes is not critical, although it is preferable to avoid the presence of compounds which may poison the catalyst. Suitable alkanes include, but are not limited to: ethane, propane, butane, pentane, cyclopentane, hexane, cyclohexane, heptane, octane, cyclooctane, decane, dodecane, tetradecane, hexadecane and mixtures thereof. As a result, the feedstock for the present process may, in addition to the alkane or alkanes of interest, further comprise methane or ethane as well as impurities such as air or carbon dioxide. It is preferred that the alkanes useful in the present invention have 2 to 12 carbon atoms, and more preferably 2 to 8 carbon atoms. Suitable preferred alkanes include, but are not limited to: ethane, propane, n-butane, isobutane, pentane, iso-pentane, hexane, iso-hexane, ethylhexane, cyclopentane, cyclohexane and mixtures thereof. Especially preferred alkanes include ethane, propane and isobutane.

As the alkanes useful in the present invention increase in carbon chain length, it is preferred that the pore size of the polyoxometallates also increase. For example. when alkanes having more than about 8 carbon atoms are used in the process of the present invention, it is preferred that the polyoxometallate supports have large pores.

Suitable oxidizing agents, or oxidants, useful in the process of the present invention include, but are not limited to: air, molecular oxygen, hydrogen peroxide, nitrogen oxides and mixtures thereof. It is preferred that the oxidizing agent is air, molecular oxygen or nitrogen oxides, and more preferably the oxidizing agent is air. The amount of the oxidizing agent useful in the present invention is any amount sufficient to oxidize the alkane. For example, the amount of oxidizing agent may be any amount up to or greater than the stoichiometric amount, based on the amount of alkane. Furthermore, by controlling the amounts of oxidizing agent and alkane, the catalyst may be kept oxidized or reduced and the lifetime and reactivity of the catalyst may be controlled.

It will be appreciated by those skilled in the art that the process of the present invention may be carried out under a variety of conditions. The conditions necessary are generally those under which the oxidizing agent functions to oxidize the alkane to an alkene. Thus, it is preferred that the process of the present invention is run under oxidizing conditions.

The process of the invention is preferably carried out in vapor phase. In the process of the present invention, an alkane, an oxidant, optionally an inert diluting gas and optionally gaseous promoters and/or modifiers are fed into a reactor. Suitable inert gases include, but are not limited to: nitrogen, argon, helium or the like. Preferably, the feedstock is an alkane gas. The alkane, oxidant and optional diluting gas may be mixed prior to being fed into the reactor or may be mixed in the reactor.

The process of the present invention may be carried out in the presence or absence of steam, and preferably in the presence of steam. When an inert, diluting gas is used in the process of the invention, determination of the molar ratio of alkane, oxidant, diluting gas and water (steam), if present, in the starting reaction gas mixture is within the ability of the skilled practitioner in the art. Determination of the gas space velocity used in the process of the invention is within the ability of the skilled practitioner in the art.

The temperature used in the process of the invention is that which favors the formation of alkenes as reaction products. The conversion of alkanes to alkenes according to the process of the present invention is carried out at a temperature in the range from 225° C to 450° C. The process of the invention is typically performed at a temperature of at least about 225° C, and preferably at least about 275° C, and below that which will cause an undesirable level of decomposition of the alkane to carbon oxide and water and/or decomposition of the catalyst. Generally, the temperature is not above 450° C, more preferably not above 400° C. Thus, a preferred temperature range is from 275° C to 400° C. It will be appreciated that when higher process temperatures are used, such as >400° C, tungsten containing catalysts are more stable and are therefore preferred for such high temperature processes. The determination of the most desirable temperature for a given reaction and given catalyst within the scope of the invention is within the ability of the person skilled in the art.

The pressure used in the process of the invention is not critical, but is important. For example, the pressure used in the present process may affect the selectivity of alkene formation. The process may be successfully carried out at atmospheric pressure. Other pressures may be used, and the determination of the most desirable pressure for a given reaction within the scope of the invention is within the ability of the person skilled in the art.

The process of the invention may be carried out in any suitable reactor configuration. For example, the reaction may be performed in a fixed-bed, moving bed, ebullating bed reactor, or other as is within the ability of the person skilled in the art to determine.

It will be appreciated that unreacted alkane from the process of the present invention may be recycled and passed through the reactor one or more times. Such a recycle has the advantage of increasing the yield of alkene produced. It will be further appreciated that the process of the present invention may be combined with a process using alkenes as feedstock, such as processes for the production of unsaturated carboxylic acids, alcohols or the like from alkenes. In such cases, the process of the present invention may be used to produce alkenes, which can then be reacted directly to form such products as unsaturated carboxylic acids, such as acrylic acid or methacrylic acid, or alcohols. In an alternative embodiment, the catalysts of the present invention may be combined with one or more additional catalysts. Such combined catalyst systems have the advantage that conversion of alkanes to alkenes and then conversion of alkenes to other reaction products may be performed within one reactor or reactor system.

The following examples are presented to illustrate further various aspects of the present invention, but are not intended to limit the scope of the invention in any aspect. All reagents were of good commercial grade and were used without further purification.

Example 1

The heteropolyacid catalysts and polyoxometallate supports were prepared according to the following general procedures.

Heteropolyacids

Phosphotungstic and phosphomolybdic acids, $H_3PW_{12}O_{40}$ and $H_3PMo_{12}O_{40}$, used in the following examples were purchased commercially.

The $H_{3+x}(PM_{12-x}V_xO_{40})$-type catalysts used in the following examples were prepared according to the following general procedure illustrated for $H_5PV_2Mo_{10}O_{40}$. $H_5PV_2Mo_{10}O_{40}$ was prepared by combining 48.8 g of $NaVO_3$ in 200 g deionized water and 14.2 g $Na_2HPO_4$ in 200 g deionized water at 95° C. After cooling the mixture, 18.4 g concentrated sulfuric acid was added at which time the solution became red and an exotherm was observed. The pH decreased from 9.1 to 3.7 with the acid addition. $Na_2MoO_4$ was then added in 400 g deionized water, followed by an additional 312 g of concentrated sulfuric acid, keeping the temperature below 50° C during the exothermic acid addition. After cooling to room temperature, 1000 ml of diethyl ether was added to the mixture with vigorous stirring. The middle layer, that is the heteropolyetherate layer, was separated and the ether was evaporated. The product was dissolved in 60-80 g deionized water and crystallized. The resulting large red crystals were filtered, washed, and dried, yielding 78.53 g of desired heteropolyacid.

The heteropolyacids with extra framework promoters were generally prepared by adding aqueous solutions (e.g. 0.1 molar) of cationic promoter species, such as $VOSO_4$, $Fe(NO_3)_3$, $Cu(NO_3)_2$, to aqueous $H_3PW_{12}O_{40}$ or $H_3PMo_{12}O_{40}$ solutions (e.g. 0.1 molar) in the appropriate amounts. The samples were heated to 60° C for 30 minutes and then the water was removed by evaporation to yield the desired, solid heteropolyacids.

Typical heteropolyacids useful in the present invention are reported in Table 1.

Table 1

| Heteropolyacids |
|---|
| $H_3PMo_{12}O_{40}$ |
| $H_3PW_{12}O_{40}$ |
| $(VO)HPMo_{12}O_{40}$ |
| $(VO)_{1.5}PMo_{12}O_{40}$ (contains $H_2SO_4$) |
| $H_5PV_2Mo_{10}O_{40}$ |
| $BiHPVMo_{11}O_{40}$ |

Polyoxometallate Supports

The polyoxometallate supports were prepared according to the following general procedure, illustrated for $Cs_3PMo_{12}O_{40}$. $Cs_3PMo_{12}O_{40}$ was prepared by adding 33.31 g of $Cs_2CO_3$ in 1225 g deionized water to 159.47 g $H_3PMo_{12}O_{40}$ in 800 g deionized water at 50° C. The addition was performed over 2 hours and the mixture was maintained at 50° C for and additional 30 minutes. After cooling to room temperature, the mixture was stirred slowly for

approximately 70 hours. The water was then removed by evaporation and the resulting solid product was dried in a vacuum oven or at elevated temperature (for example 300° C), yielding approximately 150 g of the desired polyoxometallate support.

Typical polyoxometallates useful in the present invention are reported in Table 2.

Table 2

| Polyoxometallate Supports |
|---|
| $Cs_3PMo_{12}O_{40}$ |
| $Cs_3PW_{12}O_{40}$ |
| $Cs_4PVMo_{11}O_{40}$ |
| $BiPMo_{12}O_{40}$ |

Example 2

The polyoxometallate supported heteropolyacid catalysts used in the following Examples were prepared according to the following general procedure, illustrated for the preparation of $H_3PMo_{12}O_{40}/Cs_3PMo_{12}O_{40}$.

$H_3PMo_{12}O_{40}/Cs_3PMo_{12}O_{40}$ (0.02/1.0 mole ratio - approximately 10% surface coverage) was prepared by combining 0.7 196 g $H_3PMo_{12}O_{40} \bullet 20\ H_2O$, 34.02 g $Cs_3PMo_{12}O_{40}$ and 315 g deionized water. After stirring at room temperature for 80 minutes, the water was removed by evaporation and the resulting solids were dried. The sample was heat treated at 150° C for one hour in air, yielding 32.69 g of the desired catalyst. The catalyst was then pelletized for packing in an oxidation reactor.

Examples of the catalysts prepared according to this procedure, as well as the approximate surface area of the support coated, are reported in Table 3.

Table 3

| Sample | Heteropolyacid | Polyoxometallate | POM Surface Area Covered (%) |
|---|---|---|---|
| 1 | $H_3PMo_{12}O_{40}$ | $Cs_3PMo_{12}O_{40}$ | 10 |
| 2 | $(VO)_{1.5}PMo_{12}O_{40}$ | $Cs_3PMo_{12}O_{40}$ | 10 |
| 3 | $H_5PV_2Mo_{10}O_{40}$ | $Cs_3PMo_{12}O_{40}$ | 10 |
| 4 | $H_3PMo_{12}O_{40}$ | $Cs_3PW_{12}O_{40}$ | 10 |
| 5 | $H_3PMo_{12}O_{40}$ | $Cs_3PMo_{12}O_{40}$ | 5 |
| 6 | $H_3PMo_{12}O_{40}$ | $Cs_3PMo_{12}O_{40}$ | 50 |
| C-1* | $H_3PMo_{12}O_{40}$ | $Cs_3PMo_{12}O_{40}$ | 100 |
| C-2* | $(VO)_{1.5}PMo_{12}O_{40}$ | $Cs_3PMo_{12}O_{40}$ | 100 |
| C-3* | $H_5PV_2Mo_{10}O_{40}$ | $Cs_3PMo_{12}O_{40}$ | 100 |

* Comparative

Example 3

Propane was reacted with air as the oxidizing agent and the catalysts of Example 2 in a fixed bed reactor to yield propylene. The reactor conditions and yields of propylene and acrylic acid ("AA") are reported in Table 4.

Table 4

| Sample | Reactor Temperature (°C) | Residence Time (seconds) | Propane: Air: Water | Propane Conversion (%) | AA Yield (%) | Propylen e Yield (%) | Product Selectivity (%) |
|---|---|---|---|---|---|---|---|
| 1 | 372 | 8.9 | 1:5:1.5* | 16 | 0.6 | 8.2 | 52 |
| 2 | 365 | 8.7 | 1:11:2.7 | 20 | 0.6 | 6.6 | 33 |
| 3 | 371 | 7.3 | 1:11:4.5 | 18 | 1.3 | 6.7 | 38 |
| 4 | 391 | 8.6 | 1:11:4.5 | 23 | 0.1 | 0.7 | 3 |
| 5 | 370 | 8.2 | 1:11:5.0 | 23 | 1.2 | 7.3 | 32 |
| 6 | 378 | 2.8 | 1:11:5.0 | 15 | 1.2 | 2.8 | 18 |
| C-1 | 387 | 2.7 | 1:11:5.2 | 18 | 2.0 | 0 | 11 |
| C-2 | 396 | 2.8 | 1:11:5.5 | 29 | 7.4 | 0 | 26 |
| C-3 | 394 | 8.4 | 1:11:5.4 | 66 | 8.4 | 0 | 13 |

* This run included nitrogen at 6 times the propane volumetric flow rate.

The above data clearly demonstrate that when heteropolyacid catalysts cover less than 100% of the surface area of the polyoxometallate support, alkanes are converted to alkenes with only small amounts of unsaturated carboxylic acid being produced. In contrast, when the heteropolyacid catalysts cover 100% of the surface area of the polyoxometallate support, alkanes are converted to unsaturated carboxylic acids with no propylene being produced.

**Claims**

1. A process for conversion of alkanes to alkenes comprising contacting an alkane with an oxidizing agent with a heteropolyacid on a polyoxometallate support; wherein the heteropolyacid has the formula

$$H_{(e'-bz')}G_b(X_{k'}M_{m'-x'}M^1{}_{x'}M^2{}_{n'}O_{y'})^{-e'} \quad (I)$$

wherein G is an element selected from Groups 1-16 or an oxy ion thereof; X is an element selected from Groups 3-16; M = molybdenum, tungsten or a combination thereof; $M^1$ = vanadium; $M^2$ is a transition metal different from M and $M^1$; z' = the charge on G; b = 0 to 12; k' = 1 to 5; m' = 5 to 20; x' = 0 to 6; n' =0 to 3; y' = 18 to 62; and e' is the charge of the polyoxometallate anion;
wherein the polyoxometallate support has the formula

$$C_aH_{(e-az)}(X_kM_{m-x}M^1{}_xM^2{}_nO_y)^{-e} \quad (II)$$

wherein C is a cation selected from potassium, rubidium, cesium, magnesium, calcium, strontium, barium, transition metal, actinide metal, lanthanide metal, metal oxy ion, ammonium, tetraalkylammonium, pyridinium, quinolinium, protonated aromatic amines, protonated aliphatic amines or mixtures thereof; X is an element selected from Groups 3-16 elements; M = molybdenum, tungsten or a combination thereof; $M^1$ = vanadium; $M^2$ is a transition metal different from M and $M^1$; z = the charge on C; k = 1 to 5; m = 5 to 20; n = 0 to 3; x = 0 to 6; y = 18 to 62; and e is the charge of the polyoxometallate anion; and
wherein the heteropolyacid is present in an amount such that less than 100% of the surface area of the polyoxometallate is covered.

2. The process of claim 1 wherein the polyoxometallate support comprises cesium salt of heteropolyacid having the formula $Cs_{3+x}(PM_{12-x}V_xO_{40})$, where M is molybdenum or tungsten, and x is 0 to 3.

3. The process of claim 2 wherein the polyoxometallate support comprises $Cs_3(PMo_{12}O_{40})$, $Cs_4(PMo_{11}VO_{40})$, $Cs_5(PMo_{10}V_2O_{40})$, $Cs_6(PMo_9V_3O_{40})$, $Cs_3(PW_{12}O_{40})$, $Cs_4(PW_{11}VO_{40})$, $Cs_5(PW_{10}V_2O_{40})$, $Cs_6(PW_9V_3O_{40})$ or combinations thereof.

4. The process of claim 3 wherein the polyoxometallate support comprises $Cs_3(PMo_{12}O_{40})$ or $Cs_3(PW_{12}O_{40})$.

5. The process of claim 1 wherein the heteropolyacid comprises $H_3PMo_{12}O_{40}$, $H_3PW_{12}O_{40}$, $(VO)_{1.5}PMo_{12}O_{40}$, $(VO)_{1.5}PW_{12}O_{40}$, $(TiO)_{1.5}PMo_{12}O_{40}$, $H(VO)PMo_{12}O_{40}$, $H(VO)PW_{12}O_{40}$, $H_6PV_3Mo_9O_{40}$, $H_5PV_2Mo_{10}O_{40}$, $H_5PV_2W_{10}O_{40}$, $H_4PVMo_{11}O_{40}$, $H_4PVW_{11}O_{40}$, $RhPMo_{12}O_{40}$, $BiPMo_{12}O_{40}$, $HCrPVMo_{11}O_{40}$, $HBiPVMo_{11}O_{40}$ or combinations thereof.

6. The process of claim 5 wherein the heteropolyacid comprises $H_3PMo_{12}O_{40}$ or $H_3PW_{12}O_{40}$.

7. The process of claim 1 wherein the alkane has 2 to 20 carbon atoms.

8. The process of claim 7 wherein the alkane is selected from ethane, propane, n-butane, iso-butane, pentane, iso-pentane, hexane, iso-hexane, ethylhexane, cyclopentane, cyclohexane or mixtures thereof.

9. The process of claim 1 wherein the amount of the heteropolyacid covers 0.5 to 75% of the surface area of the polyoxometallate support.

10. The process of claim 9 wherein the amount of the heteropolyacid covers 1 to 70% of the surface area of the polyoxometallate support.

11. The process of claim 10 wherein the amount of the heteropolyacid covers 3 to 50% of the surface area of the polyoxometallate support.

12. The process of claim 1 wherein the polyoxometallate support comprises cesium salt of heteropolyacid having the formula $Cs_{3+x}(PM_{12-x}V_xO_{40})$, wherein M is molybdenum or tungsten, and x is 0 to 3; the heteropolyacid comprises $H_3PMo_{12}O_{40}$, $H_3PW_{12}O_{40}$, $(VO)_{1.5}PMo_{12}O_{40}$, $(VO)_{1.5}PW_{12}O_{40}$, $(TiO)_{1.5}PMo_{12}O_{40}$, $H(VO)PMo_{12}O_{40}$, $H(VO)PW_{12}O_{40}$, $H_6PV_3Mo_9O_{40}$, $H_5PV_2Mo_{10}O_{40}$, $H_5PV_2W_{10}O_{40}$, $H_4PVMo_{11}O_{40}$, $H_4PVW_{11}O_{40}$, $RhPMo_{12}O_{40}$, $BiPMo_{12}O_{40}$, $HCrPVMo_{11}O_{40}$, $HBiPVMo_{11}O_{40}$ or combinations thereof; the alkane is selected from ethane, propane, n-butane, iso-butane, pentane, iso-pentane, hexane, iso-hexane, ethylhexane, cyclopentane, cyclohexane or mixtures thereof; and wherein the amount of the heteropolyacid covers 0.5 to 75% of the surface area of the polyoxometallate support.

13. A catalyst composition for the conversion of alkanes to alkenes comprising a heteropolyacid on a polyoxometallate support; wherein the heteropolyacid has the formula

$$H_{(e'-bz')}G_b(X_{k'}M_{m'-x'}M^1{}_{x'}M^2{}_{n'}O_{y'})^{-e'} \quad (I)$$

wherein G is an element selected from Groups 1-16 or an oxy ion thereof; X is an element selected from Groups 3-16; M = molybdenum, tungsten or a combination thereof $M^1$ = vanadium; M2 is transition metal different from M and M1; z' = the charge on G; b = 0 to 12; k' = 1 to 5; m' = 5 to 20; x' = 0 to 6; n' = 0 to 3; y' = 18 to 62; and e' is the charge of the polyoxometallate anion;
wherein the polyoxometallate support has the formula

$$C_aH_{(e-az)}(X_kM_{m-x}M^1{}_xM^2{}_nO_y)^{-e} \quad (II)$$

wherein C is a cation selected from potassium, rubidium, cesium, magnesium, calcium, strontium, barium, transition metal, actinide metal, lanthanide metal, metal oxy ion, ammonium, tetraalkylammonium, pyridinium, quinolinium, protonated aromatic amines, protonated aliphatic amines or mixtures thereof; X is an element selected from Groups 3-16; M = molybdenum, tungsten or a combination thereof; $M^1$ = vanadium; M2 is transition metal different from M and M1; z = the charge on C; k = 1 to 5; m = 5 to 20; n = 0 to 3; x = 0 to 6; y = 18 to 62; and e is the charge of the polyoxometallate anion; and
wherein the heteropolyacid is present in an amount such that less than 100% of the surface area of the polyoxometallate is covered.

14. The composition of claim 13 wherein the polyoxometallate support comprises cesium salt of heteropolyacid having the formula $Cs_{3+x}(PM_{12-x}V_xO_{40})$, where M is molybdenum or tungsten, and x is 0 to 3.

15. The composition of claim 14 wherein the polyoxometallate support comprises $Cs_3(PMo_{12}O_{40})$, $Cs_4(PMo_{11}VO_{40})$,

$Cs_5(PMo_{10}V_2O_{40})$, $Cs_6(PMo_9V_3O_{40})$, $Cs_3(PW_{12}O_{40})$, $Cs_4(PW_{11}VO_{40})$, $Cs_5(PW_{10}V_2O_{40})$, $Cs_6(PW_9V_3O_{40})$ or combinations thereof.

**16.** The composition of claim 15 wherein the polyoxometallate support comprises $Cs_3(PMo_{12}O_{40})$ or $Cs_3(PW_{12}O_{40})$.

**17.** The composition of claim 13 wherein the heteropolyacid comprises $H_3PMo_{12}O_{40}$, $H_3PW_{12}O_{40}$, $(VO)_{1.5}PMo_{12}O_{40}$, $(VO)_{1.5}PW_{12}O_{40}$, $(TiO)_{1.5}PMo_{12}O_{40}$, $H(VO)PMo_{12}O_{40}$, $H(VO)PW_{12}O_{40}$, $H_6PV_3Mo_9O_{40}$, $H_5PV_2Mo_{10}O_{40}$, $H_5PV_2W_{10}O_{40}$, $H_4PVMo_{11}O_{40}$, $H_4PVW_{11}O_{40}$, $RhPMo_{12}O_{40}$, $BiPMo_{12}O_{40}$, $HCrPVMo_{11}O_{40}$, and $HBiPVMo_{11}O_{40}$.

**18.** The composition of claim 17 wherein the heteropolyacid comprises $H_3PMo_{12}O_{40}$ or $H_3PW_{12}O_{40}$.

**19.** The composition of claim 13 wherein the amount of the heteropolyacid covers 0.5 to 75% of the surface area of the polyoxometallate support.

**20.** The composition of claim 19 wherein the amount of the heteropolyacid covers 1 to 70% of the surface area of the polyoxometallate support.

**21.** The composition of claim 20 wherein the amount of the heteropolyacid covers 3 to 50% of the surface area of the polyoxometallate support.

**22.** A process for preparing a catalyst for the conversion of alkanes to alkenes comprising combining a heteropolyacid with a polyoxometallate support; wherein the heteropolyacid has the formula

$$H_{(e'-bz')}G_b(X_{k'}M_{m'-x'}M^1_{x'}M^2_{n'}O_{y'})^{-e'} \quad (I)$$

wherein G is an element selected from Groups 1-16 or an oxy ion thereof; X is an element selected from Groups 3-16; M = molybdenum, tungsten or a combination thereof; $M^1$ = vanadium; $M^2$ is a transition metal different from M and $M^1$; z' = the charge on G; b =0 to 12; k' = 1 to 5; m' = 5 to 20; x' = 0 to 6; n' = 0 to 3; y' = 18 to 62; and e' is the charge of the polyoxometallate anion;
wherein the polyoxometallate support has the formula

$$C_aH_{(e-az)}(X_kM_{m-x}M^1_xM^2_nO_y)^{-e} \quad (II)$$

wherein C is a cation selected from potassium, rubidium, cesium, magnesium, calcium, strontium, barium, transition metal, actinide metal, lanthanide metal, metal oxy ion, ammonium, tetraalkylammonium, pyridinium, quinolinium, protonated aromatic amines, protonated aliphatic amines or mixtures thereof; X is an element selected from Groups 3-16; M is as defined above; $M^1$ = vanadium; $M^2$ is a transition metal different from M and $M^1$; z = the charge on C; k = 1 to 5; m = 5 to 20; n = 0 to 3; x = 0 to 6; y = 18 to 62; and e is the charge of the polyoxometallate anion; and
wherein the heteropolyacid is present in an amount such that less than 100% of the surface area of the polyoxometallate is covered.

**23.** A process for preparing a catalyst composition comprising a heteropolyacid on a polyoxometallate support for the conversion of an alkane to an alkene comprising treating the polyoxometallate with a strong, proton containing acid to provide a heteropolyacid on the surface of the polyoxometallate; wherein the heteropolyacid has the formula

$$H_{(e'-bz')}G_b(X_{k'}M_{m'-x'}M^1_{x'}M^2_{n'}O_{y'})^{-e'} \quad (I)$$

wherein G is an element selected from Groups 1-16 or an oxy ion thereof; X is an element selected from Groups 3-16; M = molybdenum, tungsten or a combination thereof; $M^1$ = vanadium; $M^2$ is a transition metal different from M and $M^1$; z' = the charge on G; b = 0 to 12; k' = 1 to 5; m' = 5 to 20; x' = 0 to 6; n' = 0 to 3; y' = 18 to 62; and e' is the charge of the polyoxometallate anion;
wherein the polyoxometallate has the formula

$$C_aH_{(e-az)}(X_kM_{m-x}M^1_xM^2_nO_y)^{-e} \quad (II)$$

wherein C is a cation selected from potassium, rubidium, cesium, magnesium, calcium, strontium, barium, transition metal, actinide metal, lanthanide metal, metal oxy ion, ammonium, tetraalkylammonium, pyridinium, quinolinium, protonated aromatic amines, protonated aliphatic amines or mixtures thereof; X is an element selected from Groups 3-16; M is as defined above; $M^1$ = vanadium; $M^2$ is a transition metal different from M and $M^1$; z = the charge on C; k = 1 to 5; m = 5 to 20; n =0 to 3; x = 0 to 6; y = 18 to 62; and e is the charge of the polyoxometallate anion; and
wherein the amount of the heteropolyacid is present in an amount such that less than 100% of the surface area of the polyoxometallate is covered.

**24.** The process of claim 23 wherein the strong, proton containing acid is selected from hydrochloric acid, hydrobromic acid, sulfuric acid or nitric acid.

**25.** A catalyst composition for the conversion of an alkane to an alkene prepared according to the process of claim 23.

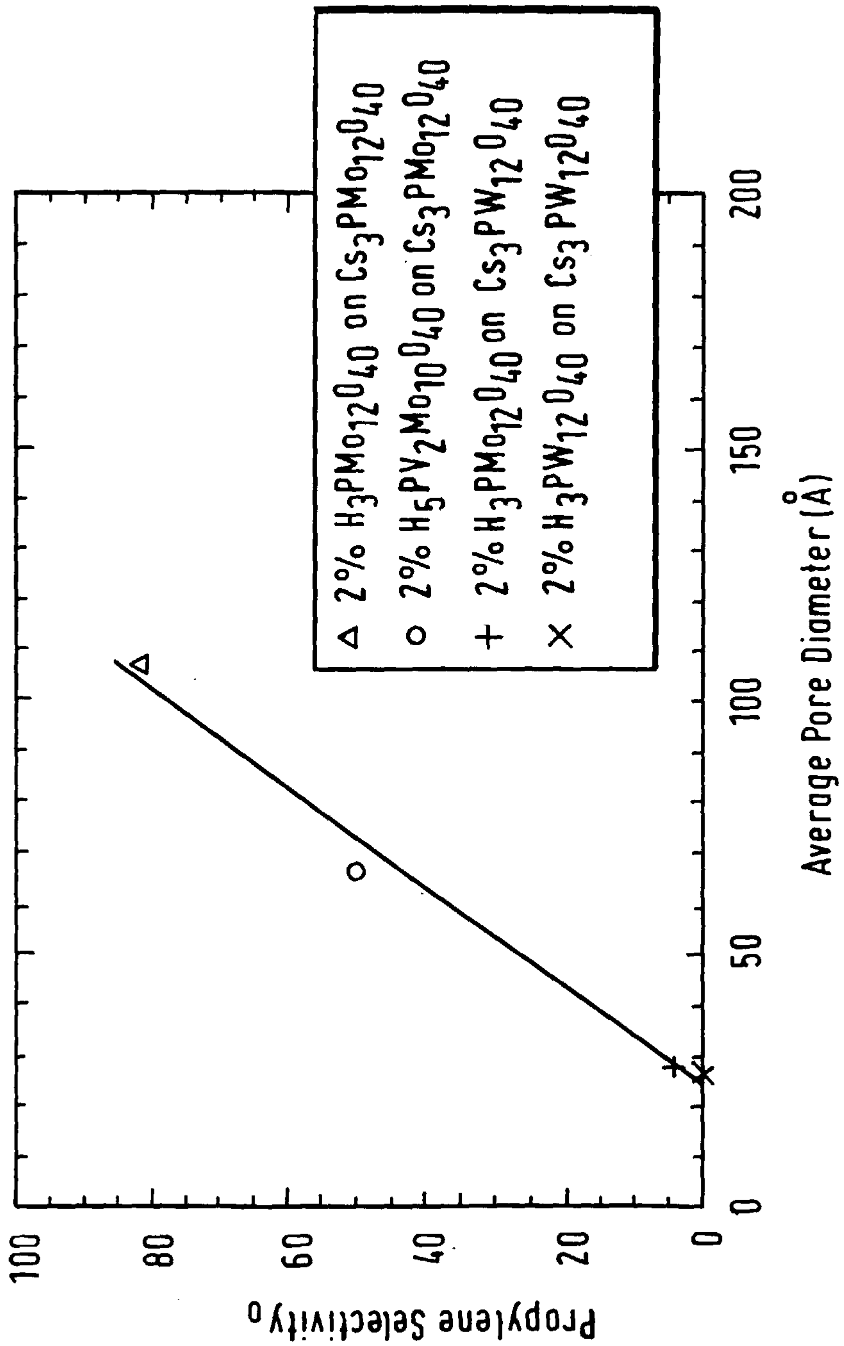

Propylene Selectivity
Average Pore Diameter (Å)
0
20
40
60
80
100
0
50
100
150
200
Δ 2% $H_3PMo_{12}O_{40}$ on $Cs_3PMo_{12}O_{40}$
o 2% $H_5PV_2Mo_{10}O_{40}$ on $Cs_3PMo_{12}O_{40}$
+ 2% $H_3PMo_{12}O_{40}$ on $Cs_3PW_{12}O_{40}$
× 2% $H_3PW_{12}O_{40}$ on $Cs_3PW_{12}O_{40}$


FIG.1.

European Patent Office

**PARTIAL EUROPEAN SEARCH REPORT**

which under Rule 45 of the European Patent Convention *shall be considered, for the purposes of subsequent* proceedings, as the European search report

Application Number EP 00 30 4571

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| P,X | WO 00 09262 A (ROHM & HAAS ;SUNOCO INC R & M (US)) 24 February 2000 (2000-02-24)<br>* claims; examples; tables * | 1-21, 23-25 | C07C5/42<br>B01J27/188 |
| P,X | US 5 990 348 A (LYONS JAMES E ET AL) 23 November 1999 (1999-11-23)<br>* claims; examples; tables * | 1-21, 23-25 | |
| A | EP 0 544 372 A (ENIRICERCHE SPA) 2 June 1993 (1993-06-02)<br>* claims * | 1 | |

**TECHNICAL FIELDS SEARCHED (Int.Cl.7)**

C07C
B01J

## INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| THE HAGUE | 11 August 2000 | Van Geyt, J |

CATEGORY OF CITED DOCUMENTS

X : *particularly relevant if taken alone*
Y : particularly relevant if combined with another document of the same category
A : technological background
O : non-written disclosure
P : intermediate document

T : theory or principle underlying the invention
E : earlier patent document, but published on, or *after the filing date*
D : document cited in the application
L : document cited for other reasons

& : member of the same patent family, corresponding document

EPO FORM 1503 03.82 (P04C07)

European Patent Office

**INCOMPLETE SEARCH SHEET C**

Application Number
EP 00 30 4571

Claim(s) searched completely:
1-21,23-25

Claim(s) not searched:
22

Reason for the limitation of the search:

Present claims 1, 13 and 23 to 25 relate to an extremely large number of possible catalyst components and oxidizing agents and their use in the conversion of alkanes to alkenes. Support within the meaning of Article 84 EPC and/or disclosure within the meaning of Article 83 EPC is to be found, however, for only a very small proportion of the catalyst components and oxidizing agents claimed. In the present case, the claims so lack support, and the application so lacks disclosure, that a meaningful search over the whole of the claimed scope is impossible. Consequently, the search has been carried out for those parts of the claims which appear to be supported and disclosed, namely those parts relating to the catalyst components mentioned in claims 3 to 6 and 14 to 18, and to the oxidizing agents listed in paragraph 2 of page 21.

Claim 22, which is an independent process claim, has not been searched as it does not contain any feature of the preparation process (Art. 84 EPC).

## ANNEX TO THE EUROPEAN SEARCH REPORT ON EUROPEAN PATENT APPLICATION NO. EP 00 30 4571

This annex lists the patent family members relating to the patent documents cited in the above-mentioned European search report.
The members are as contained in the European Patent Office EDP file on
The European Patent Office is in no way liable for these particulars which are merely given for the purpose of information.

11-08-2000

| Patent document cited in search report | Publication date | Patent family member(s) | Publication date |
|---|---|---|---|
| WO 0009262 A | 24-02-2000 | US 6060419 A | 09-05-2000 |
| US 5990348 A | 23-11-1999 | US 6060419 A | 09-05-2000 |
| EP 0544372 A | 02-06-1993 | IT 1252093 B | 01-06-1995 |

EPO FORM P0459

For more details about this annex : see Official Journal of the European Patent Office, No. 12/82